# EUROPÄISCHE PATENTSCHRIFT

(11) **EP 1 110 564 B1**
(45) Veröffentlichungstag und Bekanntmachung des Hinweises auf die Patenterteilung: **30.05.2007**
(21) Anmeldenummer: 00127879.5
(22) Anmeldetag: 20.12.2000
(51) Int. Cl.: A61M 1/28

(54) **Vorrichtung zur Peritonealdialyse**
Apparatus for the peritoneal dialysis
Appareil de dialyse péritonéale

(30) Priorität: 23.12.1999 DE 19962314
(43) Veröffentlichungstag der Anmeldung: 27.06.2001
(73) Patentinhaber: Fresenius Medical Care Deutschland GmbH, 61352 Bad Homburg v.d.H. (DE)
(72) Erfinder: Noack, Joachim, Dr., 97616 Neustadt (DE); Müller, Carsten Dr., 97302 Euerbach (DE)
(74) Vertreter: Luderschmidt, Schüler & Partner

(56) Entgegenhaltungen:
- EP-A- 0 499 718
- DE-A- 3 113 934
- US-A- 4 338 190
- US-A- 5 498 338

## Beschreibung

Die Erfindung betrifft eine Vorrichtung zur Peritonealdialyse zum Anschluß an einen oder mit einem implantierbaren Peritonealkatheters,wobei die Vorrichtung ein Überleitungsschlauchsystem und eine Einrichtung zur Bereitstellung einer Peritoneallösung aufweist.

Bei der kontinuierlichen ambulanten Peritonealdialyse (CAPD) wird über einen permanent implantierten Peritonealkatheter und ein Überleitungsschlauchsystem Peritoneallösung aus einem Folienbeutel im allgemeinen unter dem Einfluß der Schwerkraft in den Peritonealraum, d. h. die Bauchhöhle des Patienten instilliert. Das Dialysat verbleibt mehrere Stunden in der Bauchhöhle. Nach Ablauf dieses Zyklus, wird das Dialysat wieder über das Schlauchsystem abgeführt. Durch den Austausch von Flüssigkeit und gelösten Stoffen zwischen dem Blut in den peritonealen Kapillaren und dem Dialysat kann eine ausreichende Elimination hampflichtiger Substanzen erfolgen. Bei der Peritonealdialyse muß die dem Peritonealraum zugeführte Peritoneallösung absolut steril sein, da es ansonsten zu der gefürchteten Peritonitis kommen kann.

Aus der EP-A-0 149 001 ist ein Peritonealdialysegerät bekannt, das über einen Dialysator verfügt, der durch eine Membran in eine erste und zweite Kammer geteilt ist. Die erste Kammer ist über einen ersten sterilen Kreislauf mit dem Peritonealkatheter verbunden, während die zweite Kammer über einen nicht-sterilen Kreislauf mit einer Vorrichtung zur Bereitstellung von Dialysierflüssigkeit verbunden ist. Die Kreisläufe sind durch die Membran des Dialysators voneinander getrennt. An der Membran erfolgt die Abscheidung der in der Peritoneallösung enthaltenden Stoffwechselprodukte infolge Diffusion durch die Membran in die nicht-sterile Dialysierflüssigkeit.

Der Peritonealkatheter des bekannten Peritonealdialysegeräts weist zwei Lumen auf, wobei die Peritoneallösung über das erste Lumen dem Peritonealraum zugeführt und über das zweite Lumen wieder abgeführt wird.

Die US 5,498,338 beschreibt eine Vorrichtung zur Peritonealdialyse, die über einen zweilumigen Katheter verfügt. Die Peritonealdialysevorrichtung zeichnet sich dadurch aus, dass separate Mittel zur Bereitstellung frischer Peritoneallösung einerseits und zur Aufnahme von gebrauchter Lösung andererseits vorgesehen sind. Die Peritoneallösung wird mittels einer Pumpe in der Zulaufleitung über das eine Lumen des Peritonealkatheters dem Peritonealraum zugeführt und mittels einer Pumpe in der Ablaufleitung von dem Peritonealraum über das zweite Lumen des Katheters abgeführt.

Der Erfindung liegt die Aufgabe zugrunde, eine Vorrichtung zur Peritonealdialyse zu schaffen, bei der Ein- und Auslauf der Peritoneallösung beschleunigt ist, so daß die gesamte Behandlungsdauer verkürzt ist.

Die Lösung dieser Aufgabe erfolgt erfindungsgemäß mit den im Patentanspruch 1 oder 2angegebenen Merkmalen.

Bei der erfindungsgemäßen Vorrichtung zur Peritonealdialyse kann eine Strömungsverbindung zwischen der Einrichtung zur Bereitstellung und Aufnahme der Peritoneallösung aus dem Peritonealraum sowohl zu der Zulaufleitung als auch der Ablaufleitung hergestellt werden. Darüber hinaus können die Zu- und Ablaufleitung geöffnet und geschlossen werden.

Zum Befüllen des Peritonealraums wird die Ablaufleitung geschlossen und die Peritoneallösung aus der Einrichtung zur Bereitstellung derselben über die Zulaufleitung in den Peritonealraum gepumpt, während zum Entleeren des Peritonealraums die Peritoneallösung bei geschlossener Zu- und geöffneter Ablaufleitung über die Ablaufleitung in die Einrichtung zur Bereitstellung und Aufnahme der Peritoneallösung aus dem Peritonealraum zurückgepumpt wird. Eine Flussumkehr findet dabei nicht statt. Da die Förderung der Lösung sowohl beim Befüllen als auch Entleeren mittels einer Pumpe erfolgen kann, ist der Ein- und Auslauf der Lösung beschleunigt. Damit ist die gesamte Behandlungsdauer verkürzt.

Bei einer bevorzugten Ausführungsform umfassen die Mittel zum Öffnen und schließen der Zu- und Ablaufleitung ein erstes Sperrorgan in der Zulaufleitung und ein zweites Sperrorgan in der Ablaufleitung.

Eine weitere bevorzugte Ausführungsform sieht vor, daß die Mittel zur Herstellung und Unterbrechung der Strömungsverbindung zwischen der Einrichtung zur Bereitstellung und Aufnahme der Peritoneallösung aus dem Peritonealraum und der Zulaufleitung einerseits und der Ablaufleitung andererseits eine Verbindungsleitung mit einem dritten und vierten Sperrorgan umfaßt, die an einem ersten Anschlußpunkt stromauf des ersten Sperrorgans von der Zulaufleitung abgeht und an einem zweiten Anschlußpunkt stromab des zweiten Sperrorgans zu der Ablaufleitung führt. Die Mittel zur Bereitstellung und Aufnahme der Peritoneallösung aus dem Peritonealraum sind an einem dritten Anschlußpunkt zwischen dem dritten und vierten Sperrorgan an die Verbindungsleitung angeschlossen. Durch wechselweises Öffnen bzw. Schließen des dritten und vierten Sperrorgans kann die Strömungsverbindung zwischen der Einrichtung zur Bereitstellung und Aufnahme der Peritoneallösung aus dem Peritonealraum und der Zulaufleitung einerseits und der Ablaufleitung anderseits hergestellt und unterbrochen werden.

Die Mittel zur Bereitstellung und Aufnahme der Peritoneallösung aus dem Peritonealraum sind vorteilhafterweise ein Behältnis, insbesondere ein Beutel mit einem Ein-/Auslaß, der über eine Anschlußleitung mit der Verbindungsleitung verbunden ist. Über die Verbindungsleitung kann Peritonealflüssigkeit dem System sowohl zu- als auch abgeführt werden. Anstelle einer Verbindungsleitung sind aber auch zwei getrennte Leitungen zum Zu- bzw. Abführen der Lösung möglich.

Die Pumpe zur Förderung der Peritoneallösung ist vorteilhafterweise stromab des zweiten Anschlußpunkts angeordnet, an dem die Verbindungsleitung in die Ablaufleitung mündet.

Eine besonders bevorzugte Ausführungsform der Peritonealdialysevorrichtung verfügt über einen durch eine Membran in eine erste und zweite Kammer unterteilten Dialysator, wobei die Zulaufleitung mit dem Auslaß der ersten Kammer und die Ablaufleitung des Überleitungsschlauchsystems mit dem Einlaß der ersten Kammer des Dialysators verbunden ist. Der Einlaß der zweiten Kammer ist mit einer Einrichtung zur Bereitstellung von Dialysierflüssigkeit und der Auslaß der zweiten Kammer des Dialysators mit einem Ablauf verbunden. Während die erste Kammer von der Peritoneallösung durchströmt wird, fließt die Dialysierflüssigkeit durch die zweite Kammer des Dialysators, so daß an dessen Membran die Abscheidung der in der Peritoneallösung enthaltenden Stoffwechselprodukte erfolgen kann. Die über den Ablauf abfließende Dialysierflüssigkeit kann entweder entsorgt oder wieder aufbereitet werden. Beim Befüllen und Entleeren des Peritonealraums strömt die Peritoneallösung durch die erste Kammer des Dialysators in den bzw. aus dem Peritonealraum.

Die Sperrorgane sind zweckmäßigerweise elektromagnetisch oder pneumatisch betätigbare Schlauchklemmen.

Im Fall von elektromagnetisch oder pneumatisch betätigbaren Schlauchklemmen kann eine Steuereinheit vorgesehen sein, mit der die Sperrorgane derart angesteuert werden, daß die einzelnen Phasen, nämlich Befüllen, Dialyse und Entleeren automatisch vorgegeben werden.

Der Peritonealkatheter kann ein einlumiger Katheter sein. Anstelle eines einlumigen Katheters kann aber auch ein zweilumiger Katheter vorgesehen sein, wobei das erste Lumen zum Zuführen der Peritoneallösung mit der Zulaufleitung und das zweite Lumen zum Abführen der Lösung mit der Ablaufleitung verbunden ist. Unter einem zweilumigen Peritonealkatheter wird sowohl ein Katheter verstanden, der aus einem zweilumigen Schlauch besteht, als auch ein Katheter der zwei einlumige Schläuche umfaßt.

Das Schlauchsystem kann an den Katheter mittels eines Konnektors angeschlossen werden, so daß das Schlauchsystem von dem Katheter abgenommen werden kann. Die Zu- und Ablaufleitung des Überleitungsschlauchsystems können aber auch einstückiger Bestandteil des Peritonealkatheters sein.

Im folgenden wird ein Ausführungsbeispiel der Erfindung unter Bezugnahme auf die Zeichnungen näher erläutert.

Es zeigen:
- Figur 1: eine Vorrichtung zur Peritonealdialyse in vereinfachter schematischer Darstellung, wobei die Phase des Befüllens des Peritonealraumes dargestellt ist,
- Figur 2: die Peritonealdialysevorrichtung von Figur 1, wobei die Phase des Dialysebetriebs dargestellt ist und

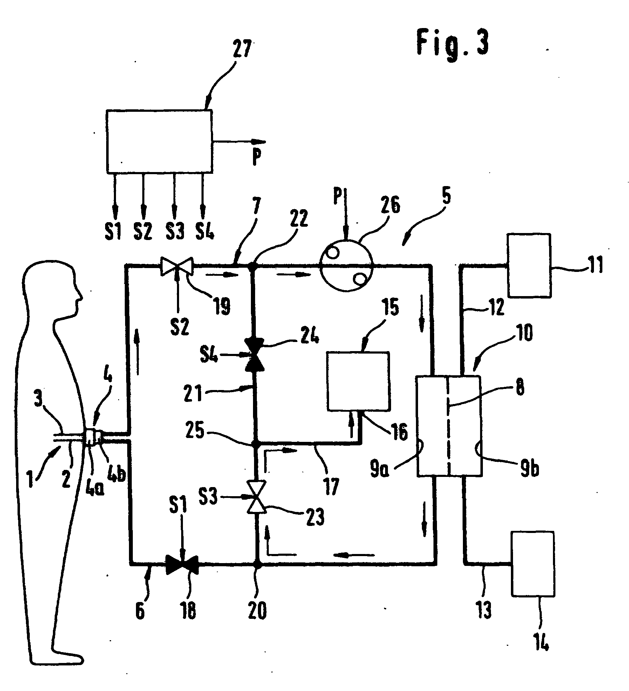

- Figur 3: die Vorrichtung von Figur 1, wobei die Phase des Entleerens des Peritonealraums dargestellt ist.

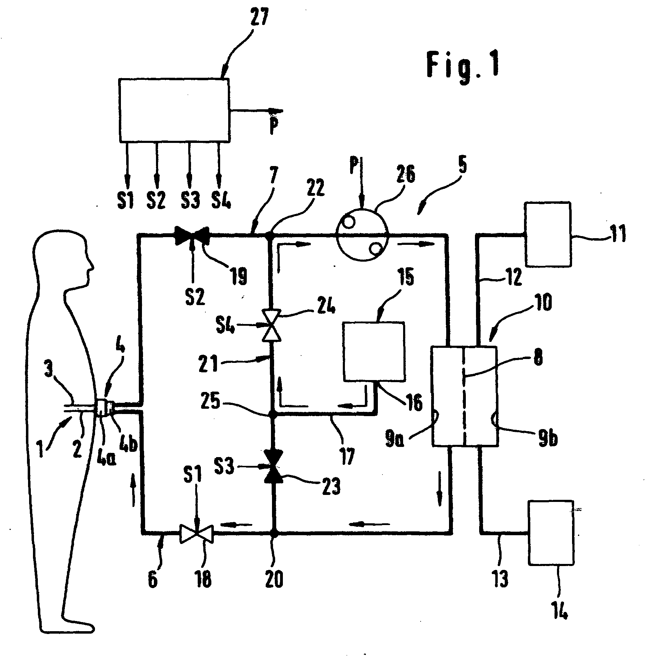

Figur 1 zeigt die wesentlichen Komponenten der Vorrichtung zur Peritonealdialyse in vereinfachter schematischer Darstellung. Die Peritonealdialysevorrichtung umfaßt einen permanent implantierbaren Peritonealkatheter 1, der ein erstes und zweites Lumen 2, 3 zum Zuführen einer Peritoneallösung in den Peritonealraum bzw. Abführen der Lösung aus dem Peritonealraum des Patienten aufweist. Derartige zweilumige Peritonealkatheter sind allgemein bekannt, so daß es keine weiteren Erläuterung bedarf.

Der Peritonealkatheter 1 ist mittels eines Konnektors 4 mit einem Überleitungsschlauchssystem 5 verbunden, das eine Zulaufleitung 6 und eine Ablaufleitung 7 aufweist. Das eine Ende der Zulaufleitung 6 ist mit dem ersten Lumen 2 des Katheters verbunden, während das andere Ende der Zulaufleitung mit dem Auslaß der ersten Kammer 9a eines durch eine semipermeable Membran 8 in die erste Kammer und eine zweite Kammer 9b unterteilten Dialysators 10 verbunden ist. Der Einlaß der ersten Kammer 9a des Dialysators 10 ist mit dem einen Ende der Ablaufleitung 7 verbunden, während das andere Ende der Ablaufleitung mit dem zweiten Lumen 3 des Peritonealkatheters 1 verbunden ist.

Der Dialysators kann Bestandteil einer herkömmlichen Dialysevorrichtung sein. Die Dialysevorrichtung verfügt neben anderen Komponenten, die der besseren Übersichtlichkeit halber hier nicht dargestellt sind, über eine Einrichtung 11 zur Bereitstellung der Dialysierflüssigkeit, die über eine Zuführleitung 12 mit dem Einlaß der zweiten Kammer 9b des Dialysators 10 verbunden ist. Der Auslaß der zweiten Kammer 9b des Dialysators 10 ist über eine Ablaufleitung 13 mit einem Auslauf 14 verbunden. Während des Betriebs strömt die frische Dialysierflüssigkeit durch die zweite Kammer des Dialysators und wird anschließend verworfen oder wieder aufbereitet.

Der Konnektor 4, der aus zwei zusammensteck- bzw. schraubbaren Teilstücken 4a, 4 b besteht, erlaubt es, das Überleitungsschlauchsystem 5 von dem permanent implantierten Peritonealkatheter 1 abzunehmen, so daß sich der Patient frei bewegen kann. Anderseits kann das Schlauchsystem auch mit dem Katheter verschweißt sein. Die reversible Trennung des Leitungssystems erfolgt dann mittels einer speziellen Schlauchschweißmaschine, die zum Stand der Technik gehört.

Die Peritoneallösung, bei der es sich auch um eine Dialysierflüssigkeit handelt, wird in einer Einrichtung 15, vorteilhafterweise einem Folienbeutel bereitgestellt. Der Beutel 15 weist einen Ein-/Auslaß 16 auf, der über eine Anschlußleitung 17 mit dem Schlauchsystem verbunden ist.

In der Zulaufleitung 6 ist ein erstes Sperrorgan 18 vorgesehen, während in der Ablaufleitung 7 ein zweites Sperrorgan (15) zum Öffnen bzw. Schließen der Zu- bzw. Ablaufleitung vorgesehen ist. Von der Zulaufleitung 6 zweigt an einem Anschlußpunkt 20 stromauf des ersten Sperrorgans 18 eine Verbindungsleitung 21 ab, die an einem Anschlußpunkt 22 stromab des zweiten Sperrorgans (19) zu der Ablaufleitung 7 führt. In der Verbindungsleitung 21 sind ein drittes Sperrorgan 23 und ein viertes Sperrorgan 24 vorgesehen. Die Anschlußleitung 17 des Beutels 15 ist an einem dritten Anschlußpunkt 25 zwischen dem dritten und vierten Sperrorgan 23, 24 mit der Verbindungsleitung 21 verbunden.

Die Förderung der Peritoneallösung erfolgt mit einer Pumpe 26, insbesondere einer okkulierenden Schlauchpumpe, die in dem Abschnitt der Ablaufleitung 7 zwischen dem zweiten Anschlußpunkt 22 und der ersten Kammer 9a des Dialysators 10 vorgesehen ist.

Bei den Sperrorganen handelt es sich um elektromagnetisch betätigbare Schlauchklemmen, die über Steuerleitungen S1 bis S4 mit einer zentralen Steuereinheit 27 verbunden sind. Anstelle von elektromagnetisch betätigbaren Schlauchklemmen können auch pneumatische Klemmen vorhanden sein. Die Ansteuerung der Pumpe 26 erfolgt von der Steuereinheit 27 über eine Steuerleitung P.

Die Peritonealdialysevorrichtung kann noch über weitere Komponenten, beispielsweise zusätzliche Pumpen zum Fördern der Flüssigkeiten, Flüssigkeitsspeicher oder Tropfkammern sowie Druckaufnehmer verfügen, die aber der besseren Übersichtlichkeit halber nicht dargestellt sind. Diese Komponenten sind beispielsweise in der EP-A-0 149 001 beschrieben, die eine bekannte Peritonealdialysevorrichtung betrifft. Es kann auch eine Vorrichtung zur Bestimmung des Beutelinhalts, beispielsweise eine Waage, vorgesehen sein, die zur Steuerung der Dialysevorrichtung mit deren Steuereinheit verbunden ist.

Die zentrale Steuerung der Peritonealdialysevorrichtung übernimmt die Steuereinheit 27, von der die Sperrorgane und die Pumpe angesteuert werden. Der Arbeitsablauf ist wie folgt.

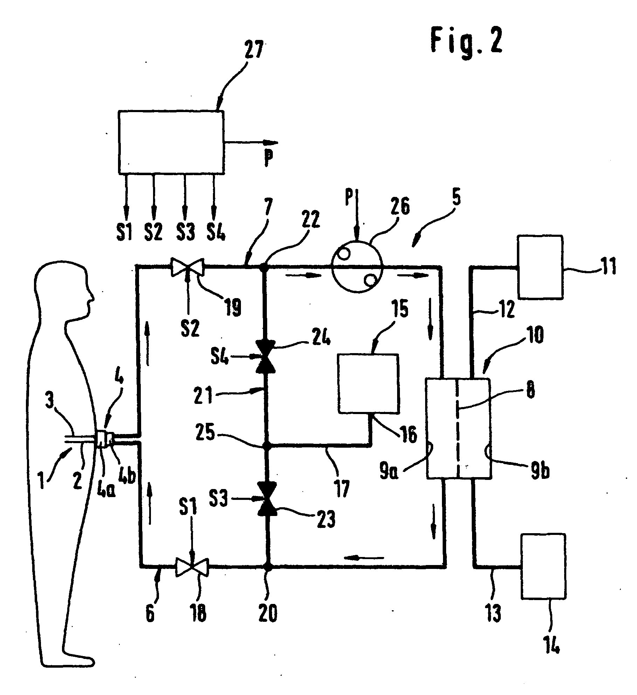

Zur Einleitung der Peritonealdialyse wird in einer ersten Phase der Peritonealraum, d. h. die Bauchhöhle des Patienten mit der Peritoneallösung befüllt. Die Steuereinheit 27 steuert die Sperrorgane hierzu derart an, daß das erste Sperrorgan 18 und das vierte Sperrorgan 24 geöffnet sind, das zweite Sperrorgan 19 und das dritte Sperrorgan 23 hingegen geschlossen sind. Die geschlossenen Sperrorgane 19,23 sind in Figur 1 schwarz unterlegt. Zum Fördern der Peritoneallösung setzt die Steuereinheit 27 die Pumpe 26 in Gang, die Peritoneallösung aus dem Beutel 15 durch die Anschlußleitung 17, die Verbindungsleitung 21, die Ablaufleitung 7, die erste Kammer 9a des Dialysators 10 und die Zulaufleitung 6 sowie das erste Lumen 2 des Peritonealkatheters 1 in den Peritonealraum fördert. Die Strömungrichtung ist in Figur 2 mit Pfeilen gekennzeichnet.

Nach Ablauf eines ersten Zeitintervals schließt die Steuereinheit 27 das vierte Sperrorgan 24 und öffnet das zweite Sperrorgan 19. In Figur 2 sind die geschlossenen Sperrorgane wieder schwarz unterlegt. Daraufhin pumpt die Pumpe 26 die Peritoneallösung über das zweite Lumen 3 des Peritonealkatheters 1 und die Ablaufleitung 7 aus dem Peritonealraum in den Dialysator 10 und aus dem Dialysator über die Zulaufleitung 6 wieder in den Peritonealraum. Die Pumpe 26 ist über ein zweites vorgegebenes Zeitinterval, das der Behandlungsdauer oder einen Bruchteil davon entspricht, in Betrieb.

Nach Ablauf der Behandlungsdauer öffnet die Steuereinheit 27 das dritte Sperrorgan 23 und schließt das erste Sperrorgan 18. Peritoneallösung wird nun mittels der Pumpe 26 aus dem Peritonealraum über die Ablaufleitung 7, den Dialysator 10, die Zulaufleitung 6, die Verbindungsleitung 21 und die Anschlußleitung 17 in den Beutel 15 zurückgepumpt.

## Patentansprüche

1. Vorrichtung zur Peritonealdialyse zum Anschluß an einen implantierbaren Peritonealkatheters (1) zum Zuführen bzw. Abführen einer Peritoneallösung aus dem Peritonealraum, mit einem Überleitungsschlauchsystem (5), das eine mit dem Peritonealkatheter verbindbare Zulaufleitung (6) und eine mit dem Peritonealkatheter verbindbare Ablaufleitung (7) aufweist, und Mitteln (18, 19) zum Öffnen und Schließen der Zu- und Ablaufleitung
**gekennzeichnet durch**
eine Einrichtung (15) sowohl zur Bereitstellung als auch zur Aufnahme einer Peritoneallösung aus dem Peritonealraum und
Mittel (23, 24) zur Herstellung und Unterbrechung einer Strömungsverbindung zwischen der Einrichtung (15) zur Bereitstellung und Aufnahme der Peritoneallösung aus dem Peritonealraum und der Zulaufleitung (6) einerseits und der Ablaufleitung (7) andererseits.

2. Vorrichtung zur Peritonealdialyse zum Zuführen bzw. Abführen einer Peritoneallösung aus dem Peritonealraum, mit einem implantierbaren Peritonealkatheter (1) und mit einem Überleitungsschlauchsystem (5), das eine mit dem Peritonealkatheter verbundene Zulaufleitung (6) und eine mit dem Peritonealkatheter verbundene Ablaufleitung (7) aufweist, und Mitteln (18, 19) zum Öffnen und Schließen der Zu- und Ablaufleitung,
**gekennzeichnet durch**
eine Einrichtung (15) sowohl zur Bereitstellung als auch zur Aufnahme einer Peritoneallösung aus dem Peritonealraum, und
Mittel (23, 24) zur Herstellung und Unterbrechung einer Strömungsverbindung zwischen der Einrichtung (15) zur Bereitstellung und Aufnahme der Peritoneallösung aus dem Peritonealraum und der Zulaufleitung einerseits und der Ablaufleitung andererseits.

3. Vorrichtung nach Anspruch 1 oder 2, **dadurch gekennzeichnet, daß** die Mittel zum Öffnen und Schließen der Zu- und Ablaufleitung ein erstes Sperrorgan (18) in der Zulaufleitung (6) und ein zweites Sperrorgan (19) in der Ablaufleitung (7) umfassen.

4. Vorrichtung nach Anspruch 3, **dadurch gekennzeichnet, daß** die Mittel zur Herstellung und Unterbrechung der Strömungsverbindung zwischen der Einrichtung zur Bereitstellung und Aufnahme der Peritoneallösung aus dem Peritonealraum und der Zulaufleitung einerseits und der Ablaufleitung andererseits eine von der Zulaufleitung (6) an einem ersten Anschlußpunkt (20) stromauf des ersten Absperrorgans (18) abgehende und an einem zweiten Anschlußpunkt (22) stromab des zweiten Sperrorgans 19 zu der Ablaufleitung (7) führende Verbindungsleitung (21) mit einem dritten und vierten Sperrorgan (23, 24) umfassen, wobei die Mittel (15) zur Bereitstellung und Aufnahme der Peritoneallösung aus dem Peritonealraum an einem dritten Anschlußpunkt (25) zwischen dem dritten und vierten Sperrorgan an die Verbindungsleitung angeschlossen sind.

5. Vorrichtung nach Anspruch 4, **dadurch gekennzeichnet, daß** die Mittel zur Bereitstellung und Aufnahme der Peritoneallösung aus dem Peritonealraum ein Behältnis (15), insbesondere einen Beutel mit einem Ein-/Auslaß (16) umfassen, das über eine Anschlußleitung (17) mit der Verbindungsleitung (21) verbunden ist.

6. Vorrichtung nach Anspruch 4 oder 5, **dadurch gekennzeichnet, daß** in der Ablaufleitung (7) stromab des zweiten Anschlußpunkts (22) eine Pumpe (26) angeordnet ist.

7. Vorrichtung nach einem der Ansprüche 1 bis 6, **dadurch gekennzeichnet, daß** die Zulaufleitung (6) mit einem Auslaß einer ersten Kammer (9a) eines durch einen Membran (8) in die erste Kammer und eine zweite Kammer (9b) unterteilten Dialysators (10) verbunden ist, und die Ablaufleitung (7) mit einem Einlaß der ersten Kammer verbunden ist, und das ein Einlaß der zweiten Kammer mit einer Einrichtung (11) zur Bereitstellung von Dialysierflüssigkeit und ein Auslaß der zweiten Kammer mit einem Ablauf (14) verbunden ist.

8. Vorrichtung nach einem der Ansprüche 4 bis 7, **dadurch gekennzeichnet, daß** eine die Sperrorgane (18, 19, 23, 24) ansteuernde Steuereinheit (27) vorgesehen ist, die derart ausgebildet ist, daß
in einem ersten Zeitintervall zum Einlauf der Peritoneallösung in den Peritonealraum das zweite und dritte Sperrorgan (19, 23) geschlossen und das erste und vierte Sperrorgan (18, 24) geöffnet werden können,
in einem zweiten Zeitintervall zur Durchführung der Dialyse das dritte und vierte Sperrorgan (23, 24) geschlossen und das erste und zweite Sperrorgan (18, 19) geöffnet werden können, und
in einem dritten Zeitintervall zum Auslauf der Peritoneallösung aus dem Peritonealraum das erste und vierte Sperrorgan (18, 24) geschlossen und das zweite und dritte Sperrorgan (19, 23) geöffnet werden können.

9. Vorrichtung nach Anspruch 8, **dadurch gekennzeichnet, daß** die Sperrorgane (18, 19, 23, 24) elektromagnetisch oder pneumatisch betätigbare Schlauchklemmen sind.

10. Vorrichtung nach einem der Ansprüche 1 bis 9, **dadurch gekennzeichnet, daß** der Peritonealkatheter (1) ein erstes Lumen (2) zum Zuführen und ein zweites Lumen (3) zum Abführen der Peritoneallösung aus dem Peritonealraum aufweist, wobei die Zulaufleitung (6) mit dem ersten Lumen und die Ablaufleitung (7) mit dem zweiten Lumen des Peritonealkatheters verbunden ist.

## Claims

1. A device for peritoneal dialysis for the connection to an implantable peritoneal catheter (1) for the supply and discharge of a peritoneal solution from the peritoneal space, with a delivery tube system (5) which has a supply line (6) connectable to the peritoneal catheter and a discharge line (7) connectable to the peritoneal catheter, and means (18, 19) for opening and closing the supply and discharge line,
**characterised by**
a device (15) both for preparing and for accommodating a peritoneal solution from the peritoneal space and
means (23, 24) for producing and interrupting a flow connection between the device (15) for preparing and accommodating the peritoneal solution from the peritoneal space and the supply line (6) on the one hand and the discharge line (7) on the other hand.

2. The device for peritoneal dialysis for the supply and discharge of a peritoneal solution from the peritoneal space, with an implantable peritoneal catheter (1) and with a delivery tube system (5) which has a supply line (6) connectable to the peritoneal catheter and a discharge line (7) connectable to the peritoneal catheter, and means (18, 19) for opening and closing the supply and discharge line,
**characterised by**
a device (15) both for preparing and for accommodating a peritoneal solution from the peritoneal space, and
means (23, 24) for producing and interrupting a flow connection between the device (1 S) for preparing and accommodating the peritoneal solution from the peritoneal space and the supply line on the one hand and the discharge line on the other hand.

3. The device according to claim 1 or 2, **characterised in that** the means for opening and closing the supply line and discharge line comprises a first shut-off element (18) in the supply line (6) and a second shut-off element (19) in the discharge line (7).

4. The device according to claim 3, **characterised in that** the means for producing and interrupting the flow connection between the device for preparing and accommodating the peritoneal solution from the peritoneal space and the supply line on the one hand and the discharge line on the other hand comprises a connecting line (21) with a third and fourth shut-off element (23, 24), said connecting line departing from the supply line (6) at a first junction point (20) upstream of the first shut-off element (18) and leading to the discharge line (7) at a second junction point (22) downstream of the second shut-off element (19), wherein the means (15) for preparing and accommodating the peritoneal solution from the peritoneal space are connected to the connecting line at a third junction point (25) between the third and fourth shut-off elements.

5. The device according to claim 4, **characterised in that** the means for preparing and accommodating the peritoneal solution from the peritoneal space comprises a container (15), in particular a bag with an inlet/outlet (16), which is connected via a junction line (17) to the connecting line (21).

6. The device according to claim 4 or 5, **characterised in that** a pump (26) is disposed in the discharge line (7) downstream of the second junction point (22).

7. The device according to any one of claims 1 to 6, **characterised in that** the supply line (6) is connected to an outlet of a first chamber (9a) of a dialyser (10) divided by a membrane (8) into the first chamber and a second chamber (9b), and the discharge line (7) is connected to an inlet of the first chamber, and that an inlet of the second chamber is connected to a device (11) for preparing dialysing fluid and an outlet of the second chamber is connected to a drain (14).

8. The device according to any one of claims 4 to 7, **characterised in that** a control unit (27) controlling the shut-off element (18, 19, 23, 24) is provided, said control unit being configured in such a way that,
in a first time interval for the inflow of the peritoneal solution into the peritoneal space, the second and third shut-off elements (19, 23) can be closed and the first and fourth shut-off elements (18, 24) can be opened,
in a second time interval for the performance of the dialysis, the third and fourth shut-off element (23, 24) can be closed and the first and second shut-off element (18, 19) can be opened, and
in a third time interval for the outflow of the peritoneal solution out of the peritoneal space, the first and fourth shut-off element (18, 24) can be closed and the second and third shut-off element (19, 23) can be opened.

9. The device according to claim 8, **characterised in that** the shut-off elements (18, 19, 23, 24) are tube clamps actuatable electromagnetically or pneumatically.

10. The device according to any one of claims 1 to 9, **characterised in that** the peritoneal catheter (1) has a first lumen (2) for the supply and a second lumen (3) for the discharge of the peritoneal solution from the peritoneal space, the supply line (6) being connected to the first lumen and the discharge line (7) being connected to the second lumen of the peritoneal catheter.

## Revendications

1. Dispositif pour une dialyse péritonéale pour le raccordement à un cathéter péritonéal (1) implantable pour l'arrivée et/ou l'évacuation d'une solution péritonéale provenant de l'espace péritonéal, comprenant un système de flexible de transfert (5), qui comporte une conduite d'entrée (6) pouvant être reliée au cathéter péritonéal et une conduite de sortie (7) pouvant être reliée au cathéter péritonéal, et des moyens (18, 19) pour l'ouverture et la fermeture de la conduite d'entrée et de la conduite de sortie,
**caractérisé par**
un dispositif (15) destiné aussi bien à la mise à disposition qu'au logement d'une solution péritonéale provenant de l'espace péritonéale et
des moyens (23, 14) pour la fabrication et la suspension d'une liaison d'écoulement entre le dispositif (15) pour la mise à disposition et le logement de la solution péritonéale provenant de l'espace péritonéal et la conduite d'entrée (6) d'une part et la conduite de sortie (7) d'autre part.

2. Dispositif pour la dialyse péritonéale pour l'arrivée et l'évacuation d'une solution péritonéale provenant de l'espace péritonéal, comprenant un cathéter péritonéal (1) implantable et un système de flexible de transfert (5), qui comporte une conduite d'entrée (6) reliée au cathéter péritonéal et une conduite de sortie (7) reliée au cathéter péritonéal, et des moyens (18, 19) pour l'ouverture et la fermeture de la conduite d'entrée et de la conduite de sortie,
**caractérisé par**
un dispositif (15) destiné aussi bien à la mise à disposition qu'au logement d'une solution péritonéale provenant de l'espace péritonéal et
des moyens (23, 24) pour la fabrication et la suspension d'une liaison d'écoulement entre le dispositif (15) pour la mise à disposition et le logement de la solution péritonéale provenant de l'espace péritonéal et la conduite d'arrivée d'une part et la conduite de sortie d'autre part.

3. Dispositif selon la revendication 1 ou 2, **caractérisé en ce que** les moyens pour l'ouverture et la fermeture de la conduite d'entrée et de la conduite de sortie comprennent un premier organe d'arrêt (18) dans la conduite d'arrivée (6) et un second organe d'arrêt (19) dans la conduite de sortie (7).

4. Dispositif selon la revendication 3, **caractérisé en ce que** les moyens pour la fabrication et l'interruption de la liaison d'écoulement entre le dispositif pour la mise à disposition et le logement de la solution péritonéale provenant de l'espace péritonéal et la conduite d'entrée d'une part et la conduite de sortie d'autre part comprennent une conduite de liaison (21) partant de la conduite d'entrée (6) en un premier point de branchement (20) en amont du premier organe d'arrêt (18) et allant en un second point de branchement (22) en aval du second organe d'arrêt (19) à la conduite de sortie (7) avec un troisième et un quatrième organes d'arrêt (23, 24), les moyens (15) pour la mise à disposition et le logement de la solution péritonéale provenant de l'espace péritonéal étant raccordés en un troisième point de branchement (25) entre le troisième et le quatrième organes d'arrêt à la conduite de liaison.

5. Dispositif selon la revendication 4, **caractérisé en ce que** les moyens pour la mise à disposition et le logement de la solution péritonéale provenant de l'espace péritonéal comprennent un récipient (15), en particulier un sachet avec une entrée/sortie (16), qui est relié par une conduite de raccordement (17) à la conduite de liaison (21).

6. Dispositif selon la revendication 4 ou 5, **caractérisé en ce qu'**une pompe (26) est disposée dans la conduite de sortie (7) en aval du second point de branchement (22).

7. Dispositif selon l'une quelconque des revendications 1 à 6, **caractérisé en ce que** la conduite d'entrée (6) est reliée à une sortie d'une première chambre (9a) d'un dialyseur (10) subdivisé par une membrane (8) en la première chambre et une seconde chambre (9b), et la conduite de sortie (7) est reliée à une entrée de la première chambre, et **en ce qu'**une entrée de la seconde chambre est reliée à un dispositif (11) pour la mise à disposition de l'équipe de dialyse et une sortie de la seconde chambre est reliée à un écoulement (14).

8. Dispositif selon l'une quelconque des revendications 4 à 7, **caractérisé en ce qu'**il est prévu une unité de commande (27) activant les organes d'arrêt (18, 19, 23, 24) qui est conçue de telle sorte que,
dans un premier intervalle de temps, le second et le troisième organes d'arrêt (19, 23) peuvent être fermés et le premier et le quatrième organes d'arrêt (18, 24) peuvent être ouverts pour l'entrée de la solution péritonéale dans l'espace péritonéal,
dans un second intervalle de temps, le troisième et le quatrième organes d'arrêt (23, 24) peuvent être fermés et le premier et le second organes d'arrêt (18, 19) peuvent être ouverts pour la réalisation de la dialyse, et,
dans un troisième intervalle de temps, le premier et le quatrième organes d'arrêt (18, 24) peuvent être fermés et le second et le troisième organes d'arrêt (19, 23) peuvent ouverts pour l'écoulement de la solution péritonéale provenant de l'espace péritonéal.

9. Dispositif selon la revendication 8, **caractérisé en ce que** les organes d'arrêt (18, 19, 23, 24) sont des pinces de flexible pouvant être actionnées de façon électromagnétique ou pneumatique.

10. Dispositif selon l'une quelconque des revendications 1 à 9, **caractérisé en ce que** le cathéter péritonéal (1) présente un premier lumen (2) pour l'arrivée et un second lumen (3) pour l'évacuation de la solution péritonéale provenant de l'espace péritonéal, la conduite d'entrée (6) étant reliée au premier lumen et la conduite d'écoulement (7) au second lumen du cathéter péritonéal.
